# DEMANDE DE BREVET EUROPEEN

(11) **EP 2 277 449 A1**
(43) Date de publication de la demande: **26.01.2011**
(21) Numéro de dépôt: 09166264.3
(22) Date de dépôt: 23.07.2009
(51) Int. Cl.: A61B 8/00, A61B 17/225, A61B 6/04, A61B 8/08, A61B 19/00, A61G 13/10

(54) **Dispositif de positionnement d'une sonde à ultrasons**

(71) Demandeur: Siemens Schweiz AG, 8047 Zürich (CH)
(72) Inventeur: Cloutot, Laurent, 8956, Killwangen (CH)
(74) Mandataire: Fischer, Michael

(57) **Abrégé**

La présente invention décrit un dispositif de positionnement d'une sonde à ultrasons (E) apte à être pilotée pour visualiser et localiser spatialement une zone limitée (C), la dite zone étant une zone in-vivo d'un sujet, comprenant :
- au moins une partie émettrice/réceptrice de la sonde est maintenue au moyen d'un support mobile (SE) disposé sous une ouverture (O) d'un plan (T) contre lequel le sujet est superficiellement appuyé, le dit support étant déplaçable au moins parallèlement audit plan,
- une cavité (CAV) est disposée sur une étendue de l'ouverture de plan et est complètement remplie d'un gel acoustique (G), de façon à ce que ledit gel assure un guide acoustique libre de tout interstice d'air sur un canal d'ultrasons entre la partie émettrice/réceptrice de la sonde et la partie superficielle d'appui du sujet sur une des faces de la cavité.

## Description

La présente invention concerne un dispositif de positionnement d'une sonde à ultrasons selon le préambule de la revendication 1.

Actuellement, un objectif majeur réside dans la visualisation de parties internes telles qu'in-vivo chez un sujet de type humain ou animal ou un mannequin dit aussi « fantôme ». En particulier, cet objectif est crucial dès lors qu'une intervention telle qu'une irradiation locale radiothérapeutique ou technologiquement équivalente en dépend afin de pouvoir localement irradier une zone limitée incluant des cellules à détruire tout en préservant de l'irradiation de tissus et cellules saines avoisinés aux cellules malsaines.

Actuellement par exemple, lors d'une séance de radiothérapie sur des cellules cancéreuses telle qu'une tumeur ou un carcinome, un dispositif annexe à l'installation de radiothérapie permet de prendre des prises de vue de type radiographique afin de visualiser et localiser ladite tumeur in-vivo dans le référentiel de l'installation de radiothérapie, ceci aux fins de contrôler que le faisceau irradiant concentre bien son énergie sur la tumeur. Ce procédé à base d'irradiation nuisible (rayons X) n'est toutefois pas sans danger pour un patient déjà affaibli par un cancer et ne permet pas une visualisation ainsi qu'une localisation d'une cible à intervalles fréquents et donc a fortiori en temps réel lors de séances d'irradiation. De tels procédés sont aussi volumineux, coûteux et nécessitent de multiples interventions humaines pour leur positionnement, leur fonctionnement et leur maintenance. Il en ressort que le pilotage d'une cible de faisceau irradiant sur une zone in-vivo lors d'un diagnostic, d'un planning ou d'une thérapie se révèle encore trop statique et donc imprécis au cas où la zone in-vivo à cibler se déplace, par exemple simplement du fait de la respiration d'un sujet.

Un but de la présente invention est ainsi de pouvoir localiser un volume de cellules in-vivo, telles que des cellules cancéreuses, de manière dynamique spatialement et temporellement. De plus, l'invention devrait présenter une aptitude à piloter aussi dynamiquement une cible (virtuelle ou non) de faisceau d'une installation de positionnement d'un appareillage annexe sur une zone in-vivo, en vue par exemple du bon planning ou suivi d'une potentielle séance d'irradiation desdites cellules tout en épargnant précisément des tissus sains périphériques.

Enfin, un problème alternatif de l'invention concerne le fait que la visualisation et la localisation d'une tumeur par exemple doit être effectuée en préservant la santé mais aussi le confort et la quiétude du patient afin de lui éviter tout stress complémentaire à un potentiel diagnostic voire une thérapie plus lourde et astreignante. Ainsi, toute irradiation invasive ou du moins nuisible, en supplément à celle liée à une possible radiothérapie ou une thérapie similaire, ainsi que l'usage de méthodes de maintien du patient, de marquage ou autre contrainte infligée au patient doit être minimisé, et idéalement éliminé.

L'invention propose ainsi un dispositif approprié à résoudre cet ensemble de problèmes et décrit au travers de la revendication 1.

Ainsi, l'invention prévoit un dispositif de positionnement d'une sonde à ultrasons apte à être pilotée pour visualiser et localiser spatialement une zone limitée, la dite zone étant une zone in-vivo d'un sujet, comprenant :
- au moins une partie émettrice/réceptrice de la sonde est maintenue au moyen d'un support mobile, en translation et si besoin en orientation, disposé sous une ouverture d'un plan contre lequel le sujet est superficiellement appuyé, le dit support étant déplaçable au moins parallèlement audit plan,
- une cavité est disposée sur une étendue de l'ouverture de plan et est complètement remplie d'un gel acoustique, de façon à ce que ledit gel assure un guide acoustique libre de tout interstice d'air sur un canal d'ultrasons entre la partie émettrice/réceptrice de la sonde et la partie superficielle du sujet en appui sur une des faces de la cavité.

En raison de l'intégration avantageuse d'une sonde à ultrasons, il est ainsi possible de visualiser et localiser la zone limitée (volume de cellules tumorales par exemple) de façon continue et en temps réel, même chez un patient lourdement malade et affaibli, car les ultrasons n'ont pas d'effets nocifs et affaiblissants connus. Si le patient bouge par rapport à une table où il est allongé, le support mobile initialement disposé pour permettre d'inclure la zone visée et son voisinage dans son champ de vision (eux-mêmes en possible mouvement, par exemple par de simples raisons respiratoire, de flux sanguin ou d'autres aspects dynamiques in-vivo) assure un déplacement de la sonde afin de toujours cadrer la zone visée dans son champ de vision. Une unique prédisposition de la sonde peut-être réglée en début d'une phase de visualisation grâce à un opérateur, afin d'assurer une visualisation et localisation sans faille a posteriori pour finalement asservir le déplacement de la sonde pour viser la zone « tracée ».

Il est à noter que le traçage spatial de cette zone par la sonde peut être mesurable métriquement dans un référentiel absolu afin par exemple de guider (de façon synchrone et également dynamique) un faisceau annexe radio-thérapeutique exactement sur la zone à irradier. En effet, les sondes à ultrasons actuelles permettent une vision bi- ou tridimensionnelle et donc, une fois que la zone limitée (tumeur) est localisée, il est possible de recalculer sa position par la simple connaissance du déplacement de la sonde dans son support mobile par rapport au plan d'appui du patient (fixe ou du moins spatialement connu dans le référentiel de l'installation annexe de diagnostic/thérapie). Ladite localisation de la zone ciblée et donc un pilotage possible d'une cible d'un faisceau d'un appareillage annexe devant être cadrée sur ladite zone ciblée est donc fort exacte spatialement et absolument dynamique temporellement.

Un ensemble de sous-revendications présente également des avantages de l'invention sous plusieurs aspects.

Des exemples de réalisation du dispositif selon l'invention sont fournis à l'aide des figures suivantes :
- Figure 1: premier mode de réalisation d'un dispositif de positionnement d'une sonde à ultrasons selon l'invention,
- Figure 2: second mode de réalisation d'un dispositif de positionnement d'une sonde à ultrasons selon l'invention.

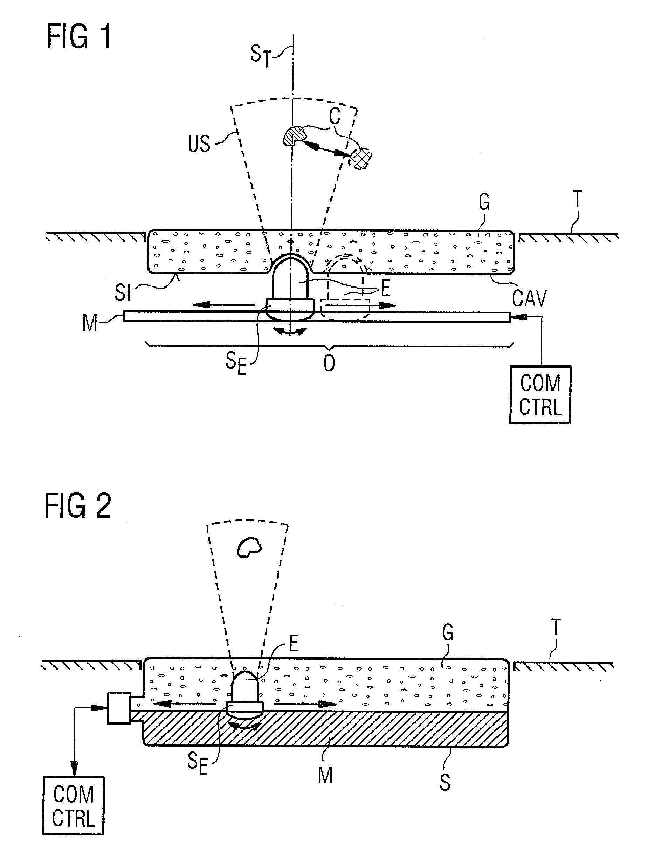

**Figure 1** présente un premier mode de réalisation d'un dispositif de positionnement d'une sonde à ultrasons (E) apte à être pilotée pour visualiser et localiser spatialement une zone limitée (C), la dite zone étant une zone in-vivo d'un sujet (tel qu'un humain ou un fantôme-mannequin), comprenant :
- au moins une partie émettrice/réceptrice de la sonde qui est maintenue au moyen d'un support mobile (SE) disposé sous une ouverture (O) d'un plan (T) contre lequel le sujet est superficiellement appuyé, le dit support étant déplaçable au moins parallèlement audit plan,
- une cavité (CAV) est disposée sur une étendue de l'ouverture de plan sous le sujet et est complètement remplie d'un gel acoustique (G), de façon à ce que ledit gel assure un guide acoustique libre de tout interstice d'air sur un canal d'ultrasons entre la partie émettrice/réceptrice de la sonde et la partie superficielle d'appui du sujet sur une des faces (supérieure) de la cavité.

A la figure 1, deux positions de la sonde E et de la zone visée C (en trait continu et en pointillé) sont représentées pour montrer un déplacement de la sonde et de son champ de mesure alors que la zone visée C se déplace également. Ce type de situation est usuel, par exemple si la zone visée C est une tumeur aux poumons qui se déplace lors des cycles de respiration d'un patient. A la figure 1, un déplacement latéral de la sonde, c'est-à-dire parallèlement à la table T, est représenté et est en pratique suffisant pour permettre de bien pouvoir visualiser et localiser spatialement et dynamiquement la zone limitée visée C. Un algorithme interne de reconnaissance et de traçage de la zone visée peut être mis en oeuvre sur un calculateur couplé au système de commande en déplacement de la sonde afin de régler les paramètres d'émission et de réception d'ultrasons de la sonde elle-même ainsi que les paramètres de son chariotage sous la table.

Le dispositif selon l'invention peut aussi prévoir que le support mobile SE soit inclinable autour d'une normale Z_{T} au plan T. Toutefois dans le cas de la figure 1, il faut alors assurer que la pénétration de la partie émettrice/réceptrice de la sonde E dans la cavité (via une paroi souple de gel) soit suffisamment importante pour éviter des effets de bord altérant l'émission et la réception de la sonde. Même si la figure 1 ne montre pas que l'axe directeur principal du champ de vision de la sonde à ultrasons E peut être toutefois légèrement incliné par rapport à la normale du plan T, il est possible par ce biais de maintenir la sonde écartée et donc protégée de toute altération et interférence provenant d'un faisceau annexe à haute énergie dont la cible devrait se focaliser sur la zone limitée C. D'autres dispositions relatives entre la sonde et le faisceau annexe peuvent être employées à ces mêmes fins, c'est-à-dire pour éviter de faire coïncider les axes principaux de la sonde et du faisceau en plus qu'un unique point.

Plus particulièrement, le dispositif selon figure 1 prévoit que la cavité présente une surface inférieure SI déformable en poussée verticale permettant ainsi une introduction (ou pénétration élastique) externe partielle de la partie émettrice/réceptrice de la sonde dans la cavité, cette introduction ayant un profil s'adaptant à un déplacement horizontal ou/et, si besoin, en orientation de la partie émettrice/réceptrice par rapport à la verticale. Dans ce cas, la surface inférieure déformable présente une impédance acoustique globale avoisinée de celle du gel, et idéalement égale à celle du gel, pour permettre un guidage optimal des ultrasons. De la même façon selon figure 1, la cavité présente une surface supérieure dont l'impédance acoustique globale est aussi avoisinée de celle du gel, et idéalement égale à celle du gel. Cette surface supérieure sera aussi idéalement souple afin de pouvoir s'adapter au profil d'une partie superficielle du sujet comme le dos d'un patient. Cela évitera tout interstice d'air à ce niveau et permettra de plus un confort accru pour le patient.

Une alternative au premier mode de réalisation du dispositif selon l'invention est présentée par la figure 2. Ici encore, la cavité a au moins une paroi traversée par les ultrasons dont l'impédance acoustique globale est avoisinée de celle du gel : il s'agit comme à la figure 1 de la paroi ou surface supérieure de la cavité. La différence avec la figure 1 réside dans le fait que la partie émettrice/réceptrice de la sonde est disposée dans la cavité, de façon à être maintenue noyée dans le gel quelque soit son déplacement ou son orientation en fonction du support mobile. Ainsi, le guidage des ultrasons se fait au travers d'une unique paroi de cavité (surface supérieure de cavité), ce qui permet une meilleure tenue d'impédance du milieu parcouru par les ultrasons par rapport à un guidage au travers de deux parois (surface inférieure et supérieure de cavité).

Cette configuration permet aussi avantageusement en plus ou au lieu de déplacer trop loin la sonde parallèlement à la table, de pouvoir incliner ou orienter sans effets de bord la sonde à l'aide d'un moyen de pivot du support mobile par rapport à la normale de la table qui sera adapté à ces fins. Ceci peut être avantageux pour éviter une trop grande surface de cavité ou de chariotage, pour visualiser des zones masquées verticalement, ainsi que pour maintenir la sonde hors du champ d'un faisceau de radiothérapie si tel est le cas.

Pour tous les modes de réalisation du dispositif selon l'invention, une unité de commande et de contrôle COM, CTRL de la sonde et du support mobile est disposée externement à la cavité et peut être reliée à ladite sonde et audit support mobile au moyen de liaisons filaires ou radio. L'unité de contrôle CTRL délivre un jeu de coordonnées spatiales de la zone visualisée dans un premier référentiel lié à la sonde, et des moyens de détermination du déplacement ou/et de l'orientation instantanée du support mobile SE délivrent ledit jeu de coordonnées dans un deuxième référentiel lié au plan T. De la sorte, une utilisation avantageuse du dispositif selon l'invention est possible en tant que moyen de localisation de la zone limitée in-vivo, pour laquelle le plan est une table sur laquelle, en tant que sujet, un être vivant est allongé ou du moins partiellement appuyé et la zone limitée est un ensemble de cellules in-vivo à localiser spatialement au moyen de coordonnées spatiales instantanément délivrées par la sonde et de moyen de calcul de la position instantanée de la sonde par rapport à un repère tridimensionnel lié au plan. A cet effet, ladite utilisation peut prévoir que la sonde est couplée à une unité de contrôle apte à une visualisation ou/et un traçage spatial de l'ensemble des cellules in-vivo, la dite unité étant pilotée par un opérateur ou/et un algorithme de reconnaissance. Etant donné que la zone limitée peut être visualisé et localisée spatialement de façon dynamiquement temporelle, l'invention présente une fort avantageuse utilisation du dispositif, pour laquelle l'unité de contrôle est reliée à une unité de commande de positionnement d'une installation de type radiothérapeutique ou équivalente dont la cible est positionnable sur la zone limitée in-vivo, par asservissement motorisé de cette cible selon les coordonnées spatiales délivrée par la sonde à ultrasons. Il est ainsi possible de rendre une radiothérapie d'une précision spatiale sans équivoque ainsi que d'une dynamique de positionnement égale à celle de la sonde à ultrasons.

## Revendications

1. Dispositif de positionnement d'une sonde à ultrasons (E) apte à être pilotée pour visualiser et localiser spatialement une zone limitée (C), la dite zone étant une zone in-vivo d'un sujet, comprenant :
- au moins une partie émettrice/réceptrice de la sonde est maintenue au moyen d'un support mobile (SE) disposé sous une ouverture (O) d'un plan (T) contre lequel le sujet est superficiellement appuyé, le dit support étant déplaçable au moins parallèlement audit plan,
- une cavité (CAV) est disposée sur une étendue de l'ouverture de plan et est complètement remplie d'un gel acoustique (G), de façon à ce que ledit gel assure un guide acoustique libre de tout interstice d'air sur un canal d'ultrasons entre la partie émettrice/réceptrice de la sonde et la partie superficielle d'appui du sujet sur une des faces de la cavité.

2. Dispositif selon une des revendications précédentes, pour lequel le support mobile (SE) est inclinable autour d'une normale (Z_{T}) au plan (T).

3. Dispositif selon revendication 1 ou 2, pour lequel la cavité a au moins une paroi traversée par les ultrasons dont l'impédance acoustique globale est avoisinée de celle du gel.

4. Dispositif selon revendication 3, pour lequel la partie émettrice/réceptrice de la sonde est disposée dans la cavité, de façon à être maintenue noyée dans le gel quelque soit son déplacement ou son orientation en fonction du support mobile.

5. Dispositif selon une des revendications 1 à 3, pour lequel la cavité présente une surface inférieure (SI) déformable en poussée verticale permettant une introduction externe partielle de la partie émettrice/réceptrice de la sonde dans la cavité, cette introduction ayant un profil s'adaptant à un déplacement horizontal ou/et en orientation de la partie émettrice/réceptrice par rapport à la verticale.

6. Dispositif selon revendication 5, pour lequel la surface inférieure déformable présente une impédance acoustique globale avoisinée de celle du gel.

7. Dispositif selon une des revendications précédentes, pour lequel une unité de commande et de contrôle (COM, CTRL) de la sonde et du support mobile est disposée externement à la cavité et est reliée à ladite sonde et audit support mobile au moyen de liaisons filaires ou radio.

8. Dispositif selon revendication 7, pour lequel l'unité de contrôle (CTRL) délivre un jeu de coordonnées spatiales de la zone visualisée dans un premier référentiel lié à la sonde, et des moyens de détermination du déplacement ou/et de l'orientation instantanée du support mobile (SE) délivrent ledit jeu de coordonnées dans un deuxième référentiel lié au plan (T).

9. Utilisation du dispositif selon une des revendications précédentes comme moyen de localisation de la zone limitée in-vivo, pour laquelle le plan est une table sur laquelle, en tant que sujet, un être vivant est allongé et la zone limitée est un ensemble de cellules in-vivo à localiser spatialement au moyen de coordonnées spatiales instantanément délivrées par la sonde et de moyen de calcul de la position instantanée de la sonde par rapport à un repère tridimensionnel lié au plan.

10. Utilisation selon revendication 9, pour laquelle la sonde est couplée à une unité de contrôle apte à une visualisation ou/et un traçage spatial de l'ensemble des cellules in-vivo, la dite unité étant pilotée par un opérateur ou un algorithme de reconnaissance.

11. Utilisation selon revendication 9 ou 10, pour laquelle l'unité de contrôle est reliée à une unité de commande de positionnement d'une installation de type radiothérapeutique dont la cible est positionnable sur la zone limitée in-vivo.
